# EUROPEAN PATENT SPECIFICATION

(11) **EP 2 156 836 B1**
(45) Date of publication and mention of the grant of the patent: **18.10.2017**
(21) Application number: 08712560.5
(22) Date of filing: 29.01.2008
(51) Int. Cl.: A61K 31/473, A61K 31/517, A61P 13/08

(54) **SYNERGIC PHARMACEUTICAL COMPOSITIONS CONSISTING OF A COMBINATION OF A 5 alpha-REDUCTASE ENZYME INHIBITOR AND A 1 alpha- ADRENERGIC RECEPTOR ANTAGONIST**
SYNERGETISCHE PHARMAZEUTISCHE ZUSAMMENSETZUNGEN AUS EINER KOMBINATION EINES 5 ALPHA REDUCTASE ENZYMHEMMERS MIT EINEM 1 ALPHA ADRENERGEN REZEPTORANTAGONISTEN
COMPOSITIONS PHARMACEUTIQUES SYNERGIQUES COMPRENANT UNE COMBINAISON D'UN INHIBITEUR DE L'ENZYME 5 alpha-RÉDUCTASE ET D'UN ANTAGONISTE DES RÉCEPTEURS 1 alpha- ADRÉNERGIQUES

(30) Priority: 02.04.2007 MX MX07003949
(43) Date of publication of application: 24.02.2010
(73) Proprietor: REPRESENTACIONES E INVESTIGACIONES MEDICAS S.A. DE C.V., Huixquilucan, Estado de Mexico (MX)
(72) Inventor: GARCÍA ARMENTA, María Elena, Zapopan, Jalisco (MX); SANTOS MURILLO, Josefina, Zapopan, Jalisco (MX); ALVAREZ OCHOA, Victor Guillermo, Zapopan, Jalisco (MX)
(74) Representative: Carvajal y Urquijo, Isabel
(86) International application number: PCT/MX2008/000012
(87) International publication number: WO 2008/120967

(56) References cited:
- WO-A1-01/21167
- WO-A1-92/16213
- US-A- 6 046 183
- DATABASE MEDLINE [Online] US NATIONAL LIBRARY OF MEDICINE (NLM), BETHESDA, MD, US; May 1998 (1998-05), TKACHUK V N ET AL: "[Combined use of 5 alpha-reductase inhibitors and alpha-l adrenergic receptor blockers in patients with benign prostatic hyperplasia]", XP002613596, Database accession no. NLM9644979
- MACDONALD RODERICK ET AL: "Alfuzosin for treatment of lower urinary tract symptoms compatible with benign prostatic hyperplasia: a systematic review of efficacy and adverse effects.", UROLOGY OCT 2005 LNKD- PUBMED:16230138, vol. 66, no. 4, October 2005 (2005-10), pages 780-788, XP005117695, ISSN: 1527-9995
- KIRBY R.S. ET AL.: 'Efficacy and tolerability of doxazosin and finasteride, alone or in combination, in treatment of symptomatic benign prostatic hyperplasia: the prospective european doxazosin and combination therapy (predict)' UROLOGY vol. 61, no. 1, 2003, pages 119 - 126, XP002462574
- KAPLAN S. ET AL.: 'The superior beneficial effect on combination therapy with doxazosin and finasteride versus either drug alone on clinical progression of benign prostatic hyperplasia in the MTOPS trial was not dependent on patients having prostatic enlargement at baseline' EUROPEAN UROLOGY no. 3, SUPPL. 4, 2005, page 213, XP005007302
- DEBRUYNE F.M.J. ET AL.: 'Sustained-release alfuzosin, finasteride and the combination of both in the treatment of benign prostatic hyperplasia' EUROPEAN UROLOGY vol. 34, 1998, pages 169 - 175, XP008119367
- CLARK R. ET AL.: 'The dual 5 alpha-reductase inhibitor dutasteride is well tolerated and has no efffect on the pharmacokinetic profiles of transulosin or terazosin, when used in combination in healthy male volunteers' EUROPEAN UROLOGY vol. 3, no. SUPPL. 4, 2005, page 212, XP005007301

## Description

### FIELD OF INVENTION

The present invention has been developed to be applied in pharmaceutical industry and describes a pharmaceutical composition composed by a synergic combination of a 5 α-reductase enzyme inhibitor agent such as Finasteride and a 1 α-adrenergic receptor antagonist agent such as the active principle Alfuzosine besides pharmaceutical acceptable excipients, which is formulated in a single dosage unit for use in Benign Prostatic Hyperplasia (BPH) and the relief of the symptoms associated with said disease.

### BACKGROUND OF THE INVENTION

Benign Prostatic Hyperplasia (BPH) is a chronic and progressive disease which affects to a large number of men as age comes.

HPB physiopathology has been attributed to an obstruction of the urine bladder output originated by a growth of prostatic gland (prostate) caused by a relative increase in estrogen production regarding to testosterone production.

The main etiologic factors which regulate or produce prostate growth in BPH are the androgenic factor constituted by the presence of dihydrotestosterone (DTH) and that concerning age. Said factors are both intrinsic and extrinsic. Epithelial, stroma and extracellular matrix cells comprising the prostate interact among them as intrinsic factors to regulate prostate growth through molecules such as epidermal growth factor (EGF), fibroblast growth factor (FGF2) and growth factor similar to insulin (IGF-I). Growth factors cause about 80% of prostate growth positive modulation. They are also considered as intrinsic factors those of genetic, systemic and testicular origin. Extrinsic or environmental factors are mediated by those intrinsic to regulate among them the prostate growth. As to the age factor, it results contradictory somehow that when testicular function begins to decline about an age of 40 years, the prostate starts hyperplasia process. Similarly, there are other extrinsic factors such as food where there are a large number of compounds which have been examined and proved that they are a cause of BPH manifestation. Fat-rich diets increase the production of serum prolactin which has a proliferative effect over prostate epithelium. On the other hand, regular alcohol consumption may reduce the risk of prostate surgery since alcohol reduces serum testosterone concentration.

Prostate is androgen-dependant for its growth and functional and structural integrity. Androgens such as testosterone and its active metabolite dihydrotestosterone (DHT) play a very important role in BPH pathogenesis. Testosterone molecule is metabolized to DHT in a reaction catalyzed by 5 α-reductase enzyme. There are two isoenzymes: type I, 5 α-reductase expressed in skin and liver and type II, 5 α-reductase which predominates in prostate. Both isoenzymes act over testosterone when in circulation. Testosterone and DHT stimulate the stromal cell growth in prostate.

BPH diagnosis is performed by taking into account the following issues which determine a clinical profile in patients: symptomatology, prostatic growth and degree of urinary obstruction. These issues vary from patient to patient since there are some who manifest symptoms with or without prostatic growth and they may have or not urine output flow obstruction.
The symptoms which are commonly manifested in BPH include in addition to prostate enlargement, increased residual urine in the bladder and the increase of prostate-specific antigen (PSA) concentration, urinary urgency, urinary frequency, nicturia, night incontinency, pain, terminal drop, decrease in urinary jet gauge and force, prolonged or paused emptiness and they are shown in most of men with more than 60 years old having thus a great impact in the quality of life.

The main therapeutics objective is to promote an improvement in urinary symptoms and urinary flow. Treatment choice for a patient suffering from BPH depends on how uncomfortable are the urinary symptoms, the severity thereof, the amount of residual urine handled by the patient and an objective demonstration of the damage caused by the manifested obstructive process.

Traditional surgical treatment is prostate transurethral resection (RTUP); however, therapeutic alternatives have been focused currently in pharmacologic treatment to reduce the prostate size and to improve the lower urinary tract symptoms.

The most commonly used medicaments in BPH pharmacological treatment are two types: 5 α-reductase enzyme inhibitors and 1 α-adrenergic receptor blockers.

5 α-reductase enzyme inhibitors reduce the dihydrotestosterone concentration (DHT) in blood and prostatic gland. DHT is a male hormone which is important in normal and abnormal growth from prostate gland, performing a key role in prostate non-cancerous growth (benign prostate enlargement or BPH) and it is also involved in developing prostate cancer. If the prostate grows too large, the urine flow can be slowed or stopped.

In BPH problem and symptoms that accompany the disease, 5 α-reductase enzyme inhibitors act by relaxing the smooth muscle in prostate walls and at bladder neck , thus resulting in a decrease of prostate size which helps reduce the pressure over urethra thus easing urine flow and the relief of lower urinary tract symptoms. They are also used for high blood pressure problems by its vasodilator effect.

The pharmacological activity of 5 α-reductase enzyme inhibitors reduce prostate size by up to 50%, an increase of apoptotic index of prostate cells, reducing the overexpression of bcl-2 gene which prevents apoptosis, in addition to improving obstructive symptomatology.

Among the active ingredients classified as 5 α-reductase enzyme inhibitors is Finasteride, which has proved effective in inhibiting the conversion of testosterone into dihydrotestosterone (DHT), thereby reducing the size of the prostate.

Finasteride is a specific inhibitor which acts by blocking 5 α-reductase enzyme action, thus preventing that the conversion of testosterone male hormone in a highly active intermediary metabolite called dihydrotestosterone (DHT) is carried out, thus providing as a result a reduction in DHT concentration in blood and in prostate gland until 30% in patients who suffer BPH.

Finasteride use reduces up to 50% of prostate-specific antigen (PSA) serum levels in patients who suffer BPH, being normal to find it in high levels in semen and low levels in blood. Incidence of adverse effects during Finasteride treatment is very low (about 3%), the following being the most common; erectile dysfunction and decrease in ejaculated semen volume.

This drug substance causes few adverse effects; however, it demands a treatment of almost one year for a fully action and release of the symptoms caused in patients with BPH. Prostate bleeding is frequently caused in patients suffering from BPH. This effect is mediated by androgen-dependant angiogenic growth factors such as endothelial vascular growth factor. Finasteride has been successfully used to control this haematuria.

1α-adrenergic receptor blockers are classified as: non-selective alpha, such as the drug substances: Doxazosine, Prazosine, Terazosine; and alpha selective, such as those in drug substances: Alfuzosine and Tamsulosine.

Urinary flow reduction and obstructive symptoms showed during urinary emptiness in patients suffering BPH are generally caused by two main factors, one is prostate growth which is the static factor and the other is the prostatic stroma and urethra smooth muscle tone, which constitute the dynamic factor. The action mechanism of these agents is the smooth muscle relaxation in bladder neck, prostate and prostatic capsule caused by a blockage performed at 1α-adrenergic receptors causing a decrease in blood pressure and urethral resistance, thus allowing reduction and/or relief of lower urinary tract symptomatology.

The effect that 1α-adrenergic receptor blockers have in smooth muscle depends on neurotransmitter release, such as noradrenaline which proceeds from adrenergic nerve terminations found in stroma and urethral smooth muscle. Irritating symptoms have been associated with a certain degree of vesical dysfunction causing urinary output flow obstruction. 1α-adrenergic receptor blockers act reducing and/or improving the lower urinary tract symptoms, being the main symptom: urinary flow output obstruction. Adrenergic receptors in urethra and prostate intervene in the dynamic components and it has been demonstrated that there is a direct relationship between the amount of smooth muscle and the obstruction in urinary flow output, thus being logical to assume that these receptors intervene at least in some degree, in the genesis of the lower urinary symptoms.

The main side effects are caused as a result of 1α-adrenergic blockage which is performed in the brain and in the cardiovascular system. Side effects which are most commonly present in about 10 to 15% of patients when administered these kind of drugs are the following: tiredness, dizziness, cephalea, sexual impotence, being less common the appearance of symptoms such as: asthenia, palpitations, digestive disorders, nausea, diarrhea, constipation, drowsiness and insomnia.

The best knowledge of different types of 1α-adrenergic receptors (such as subtypes: α-1A, α-1B, α-1D, α-1L) allowed the development of the α-blocker second generation, such as: Indoramine, Terazosine, Doxazosine, Alfuzosine and Tamsulosine. All the α-blockers have a similar efficacy but they show differences as to side effects.

Alfuzosine is a quinazoline derivative. It is a 1α-adrenergic receptor selective antagonist. They act preventing the urinary flow obstruction through the relaxation of prostatic smooth muscle and vesical neck (bladder) stress. Said relaxation is mediated by the activation of 1α-adrenergic receptors which stimulate smooth muscle contraction causing an increase in prostate tone, prostatic capsule, prostatic urethra and bladder base, thus increasing the resistance to urine passage and leading to urinary obstruction and very probably to secondary vesical instability. 1α-adrenergic receptor blockage releases the intravesical obstruction through a direct action over prostatic muscle, thus allowing that urine flows more easily.

Alfuzosine is used to treat BPH symptoms , which may be manifested in two types: Obstructive such as weak stream, difficulty in starting urination, intermittent urination, incomplete vesical evacuation and terminal dropping; and irritating such as: increase in morning urinary frequency, nicturia, urinary urgency, disuria and incontinence by urinary urgency.

Alfuzosine has a fast action start from administering the first dose since it improves the urinary flow along an one-week term, thus reducing the risk to present an urine acute retention causing a need of surgery.

In the last decade, pharmacological treatment prescription for Benign Prostatic Hyperplasia (BPH) has decreased the number of surgical procedures worldwide and there is a trend to increase in the coming years.

It has been described in the prior art 5α-reductase inhibitors and 1α-adrenergic receptor antagonists for treating HPB, as described in the patent documents WO 92/16213 A1 and US 6,046,183. In said documents, it is revealed an effective method of treatment for men with HPB including the combination therapy of a 5α-reductase inhibitor in particular Finasteride with a 1α-adrenergic receptor antagonist particularly Terazosin. They also describe pharmaceutical compositions comprising therapeutically effective amounts of active ingredients mentioned above, useful for the treatment of BPH, as well as relief of symptoms associated with the disease.

Also, there are also clinical studies in which the effectiveness of monotherapy and combination therapy among Finasteride and Alfuzosin was evaluated. Such studies have proved be controversial in relation to the reported results.

In this regard, it is published a clinical study in the U.S. National Library of Medicine (NLM), Bethesda, MD, U.S., May 1998 (1998-2005); Tkachuk, VN et al., entitled "Combined use of 5 alpha-reductase inhibitors and alpha-1 adrenergic receptor blockers in Patients with Benign Prostatic Hyperplasia", which aimed to assess the efficacy of the combination of an inhibitor of 5 α-reductase a blocker of α-adrenergic receptors 1.

For the study, it was considered 88 patients with BPH and micturition disorders, daytime and nighttime pollakiuria and urinary flow reduction who received concomitantly a 5 α-reductase inhibitor, finasteride (Proscar) and a 1 α adrenergic receptor uroselective blocker, Alfuzosin (Dalfaz). The drugs were prescribed simultaneously for 12 months. The Finasteride dose was 5 mg once a day and 5 mg Alfuzosin twice a day. In order to evaluate the treatment outcome, it was considered the evolution of the clinical picture of each patient with respect to the criteria of quality of life, micturition parameters, prostate volume, amount of residual urine in the bladder and concentration of prostate specific antigen (PSA) as well as tolerance to drugs and blood pressure. This study found that combination therapy works better than monotherapy.

On the other hand, the scientific paper entitled "Alfuzosin for Treatment of lower urinary tract Symptoms compatible with benign prostatic hyperplasia: a Systematic Review of Efficacy and Adverse Effects" Roderick Macdonald et al., Urology, Oct. 2005, vol. 66, no. 4, October 2005 (2005-10), pages 780-788, describes the carrying-out of a meta-analysis of 11 randomized trials aimed at evaluating the efficacy and safety (adverse effects) of Alfuzosin for treatment of lower urinary tract symptoms (LUTS) associated with benign prostatic hyperplasia (BPH). The study compared Alfuzosin vs. Finasteride, and the combination of both active agents. The dose was 5 mg finasteride and the one of sustained release (SR) was 10 mg Alfuzosin. From the results obtained in this study, it is concluded that Alfuzosin SR alone and in combination with finasteride was significantly more effective than Finasteride monotherapy in improving lower urinary tract symptoms assessed by the IPSS (International Prostate Symptom Score). Therefore, the Alfuzosin SR alone and the combination therapy of Finasteride + Alfuzosin, manifest similar therapeutic activity.

In turn, the scientific paper entitled "Sustained-Release Alfuzosin, Finasteride and the Combination of Both in the Treatment of Benign Prostatic Hyperplasia", Debruyne, FMJ et al., European Urology 1998, vol. 34, pages 169-175, discloses a randomized, double-blind, multicenter clinical study, in which the objective was to assess the additive benefit of combining a 1 α-adrenergic receptors blocker and 5 α-reductase inhibitor. Patients received 5 mg Alfuzosin sustained release (SR) twice a day, 5 mg Finasteride once a day, or both drugs for 6 months. The performance criteria were symptomatic improvement assessed using the International Prostate Symptom Store: I-PSS and maximum flow rate (Qₘₐₓ). Safety was assessed by monitoring adverse effects. This study concluded that Alfuzosin SR has a efficacy significantly better than Finasteride in relieving the lower urinary tract symptoms (LUTS) associated with BPH, with no additional benefit observed for combination therapy.

In an analysis of six clinical studies, Finasteride showed to be very effective when prostate weighed >40 g (Logan YT, Belgeri MT; Am J Geriatric Pharmacother, 2005 Jun; 3(2):103-14).

Unlike the present invention, combinations of active ingredients Finasteride and Alfuzosin described in the scientific papers cited above were prescribed simultaneously or concurrently in separate dosage units also it was administered to patients, known concentrations in the state of the art as effective, ie, 5 mg Finasteride and 10 mg Alfuzosin daily for long periods of time, requiring the use of treatment for about 12 months to observe significant improvement in the relief of lower urinary tract symptoms.

At present there is a wide variety of medicaments that come in various dosage forms that are prescribed for the treatment of BPH, which have independently formulated a of 5 α-reductase enzyme inhibitor agent or a 1 α-adrenergic receptor blocking agent, they are administered separately or even simultaneously.

However and despite the medical community has prescribed as a common practice for the treatment of BPH, the combination of 5 α-reductase inhibitors such as finasteride and 1α-adrenergic receptors antagonists as Alfuzosin, it has been shown that the fact to administer them separately or simultaneously causes inconvenients such as the need to increase the daily dose of 1 α-adrenergic receptor antagonist agent, the administration of higher concentrations of active ingredients, the prescription and consumption of those active ingredients for long periods of time in order to demonstrate significant relief of BPH and its symptoms; the administration of Finasteride and Alfuzosin in separate dosage units, which causes little to no adoption to treatment in patients with BPH and its symptoms, and the increase of adverse effects, reason why there is a high percentage of patients who must discontinue the treatment.

There is also controversy regarding the effectiveness of monotherapy and combination therapy with Finasteride and Alfuzosin when administered separately or simultaneously.

That is why there is still a need to develop novel pharmaceutical compositions comprising a 5 α-reductase enzyme inhibitor and a 1α-adrenergic receptors antagonist for use in the treatment of BPH and relief of symptoms that accompany the disease that show more effectiveness in less time, and therapeutic safety, and also succeed in reducing the adverse effects associated with its use.

### DETAILED DESCRIPTION OF THE INVENTION.

Therefore, considering the existing defects in the prior art, the objective of the present invention is the development of a pharmaceutical composition comprising the synergistic combination of a 5 α-reductase enzyme inhibitor, finasteride, and a 1α- adrenergic receptor antagonist, Alfuzosin, as well as pharmaceutically acceptable excipients, formulated in a single dosage unit for use in the treatment of benign prostatic hyperplasia (BPH) and the relief of symptoms accompanying said disease.

Thus, the present invention relates to a pharmaceutical composition comprising the synergistic combination of Finasteride and Alfuzosin, as well as pharmaceutically acceptable excipients, formulated in a single dosage unit to be administered by oral route.

Said pharmaceutical composition is formulated to be administered orally, preferably in tablet form.

In the pharmaceutical composition object of the present invention, the active ingredient Finasteride is present in the formulation at a preferred concentration of 5.0 mg. and the active ingredient Alfuzosin is preferably found in a concentration of 5.0 mg. per dosage unit.

Also, in the present invention it is achieved the coexistence of the active ingredients Finasteride and Alfuzosin in a same dosage unit, thus getting an unexpected synergistic effect occurs when formulated together as opposed to when they are administered separately or simultaneously, besides obtaining significant advantages such as the incorporation of lower concentrations of formulated active ingredients, the potentiation of the therapeutic effect, the decrease of adverse effects associated with the separate consumption of these active ingredients, the significant relief of BPH and the symptoms that accompany said disease in less time and better tolerance.

On the other hand, it was carried out a comparative clinical study between the pharmaceutical composition subject of the present invention and the active ingredients Finasteride, Alfuzosin, and Placebo administered separately. The results of said clinical study showed that administration of lower concentrations of the 1α-adrenergic receptors antagonist, Alfuzosin, combined with the 5α-reductase inhibitor, finasteride, produce an unexpected synergistic effect and a significant efficacy and therapeutic safety.

In this clinical trial several tests were performed wherein Placebo, Finasteride, Alfuzosine and a combination of Finasteride/Alfuzosine were administered to a population of patients diagnosed with Benign Prostatic Hyperplasia.

Said clinical trial was applied in 160 male patients with a 60-year old average, wherein the symptoms manifested according to Urology American Association (AUU), where ranking were scored (score is assigned according to the following criteria: from 0= No symptoms to 35= severe symptoms. Maximum urinary flow was then assessed with an average of 4 to 15 mL per second and emptiness volume of at least 125 mL. Finally, vital signs, treatment compliance and side effects were assessed for each of the groups.

At the beginning and at the end of said clinical trial the following examinations were applied to the patients: digital rectal examination, prostate-specific antigen (APE) assessment and urine analysis.

Exclusion Criteria:
- Patients who have had a medical or surgical intervention for BPH treatment.
- Patients with blood pressure <90/70 mm Hg in supine position.
- Patients with prostate-specific antigen levels <10 ng/mL.

Subjects were randomized and assigned to a double-blind study divided into 4 groups. Randomization was stratified and medicament administration at night.
Group 1 Placebo: Patients received a daily tablet along 12 months.
Group 2 Finasteride: Patients received a daily 5 mg tablet along 12 months.
Group 3 Alfuzosine: Patients received a daily 10 mg tablet along 12 months.
Group 4 Combination: Patients received a tablet with a combination of Finasteride 5 mg and Alfuzosine 5 mg daily along 12 months.

Obtained Results:

**Table 1**

| Base Line Characteristics of patients with BPH (n=160) | | | | | |
|---|---|---|---|---|---|
| Charact. | All | Placebo | Alfuzosine | Finasteride | |
| Combination | | | | | |
| | (n=160) | (n=40) | (n=40) | (n=40) | (n=40) |
| Average age | 64.5 ± 8.3 | 61.7 ± 8.0 | 65.3 ± 8.2 | 62.8 ± 7.4 | 66.5 ± 7.1 |
| Median | 64.0 | 63.0 | 63.0 | 62.0 | 64.0 |
| AAU Symptoms | | | | | |
| Average | 17.2 ± 6.3 | 16.7 ± 6.1 | 16.3 ± 5.3 | 17.6 ± 5.6 | 16.7 ± 5.1 |
| Median | 17.0 | 17.0 | 17.0 | 17.0 | 16.0 |
| Prost. Vol. x ml | | | | | |
| Average | 36.2 ± 20.1 | 35.1 ± 18.0 | 37.8 ± 22.0 | 37.6 ± 22.0 | 35.8 ± 17.0 |
| Median | 31.0 | 31.0 | 31.0 | 32.0 | 31.0 |
| Urine Max. Flow | | | | | |
| Average | 15.0 ± 3.3 | 15.1 ± 3.2 | 15.5 ± 3.1 | 15.4 ± 3.0 | 15.4 ± 3.1 |
| Median | 11.0 | 11.0 | 11.0 | 11.2 | 11.3 |
| Residual Vol | | | | | |
| Average | 65.1 ± 82.0 | 66.4 ± 80.0 | 67.6 ± 82.0 | 63.1 ± 80.0 | 64.0 ± 80.0 |
| Median | 38.3 | 38.1 | 40.0 | 39.0 | 38.0 |
| APE (ng/mL) | | | | | |
| Average | 2.1 ± 2.0 | 2.2 ± 2.1 | 2.3 ± 2.0 | 2.1 ± 2.0 | 2.1 ± 2.0 |
| Median | 1.5 | 1.7 | 1.7 | 1.5 | 1.5 |
| Serum creatinine | | | | | |
| Average | 1.0 ± 0.1 | 1.1 ± 0.1 | 1.0 ± 0.1 | 1.1 ± 0.1 | 1.1 ± 0.1 |
| Median | 1.0 | 1.1 | 1.0 | 1.0 | 1.0 |

| | | | | | |
|---|---|---|---|---|---|
| - AAU Symptoms: American Association of Urology - Prostate Volume per mL - Maximum Urinary Flow (mL x sec) - Post-emptiness Residual volume x mL - Prostate-Specific Antigen (ng x mL) - Serum creatinine (mg x dL) | | | | | |

There were not significant base line features among groups (Table 1).

**Table 2**

| Clinical Progression of BPH at 12 months of treatment | | | |
|---|---|---|---|
| Placebo 23/40 57.5% | Alfuzosine 18/40 45% | Finasteride 13/40 32.5% | Combination 7/40 17.5% |

| Increase in symptoms according to the score from American Association of Urology at 12 months of treatment | | | |
|---|---|---|---|
| Placebo 40/40 100% | Alfuzosine 19/40 47.5% | Finasteride 15/40 37.5% | Combination 5/40 12.5% |

| Urinary Retention | | | |
|---|---|---|---|
| Placebo 30/40 75% | Alfuzosine 21/40 52.5% | Finasteride 17/40 42.5% | Combination 4/40 10% |

| Incontinence | | | |
|---|---|---|---|
| Placebo 6/40 15% | Alfuzosine 4/40 10% | Finasteride 3/40 7.5% | Combination 1/40 2.5% |

| Urinary Tract infection | | | |
|---|---|---|---|
| Placebo 2/40 5% | Alfuzosine 2/40 5% | Finasteride 1/40 2.5% | Combination 1/40 2.5% |

| Invasive Therapy due to BPH | | | |
|---|---|---|---|
| Placebo 5/40 12.5% | Alfuzosine 4/40 10% | Finasteride 2/40 5% | Combination 1/40 2.5% |

### Side Effects

Those more frequently present common side effects are manifested in Alfuzosine group 3, compared with other groups these events included: dizziness, postural hypotension, asthenia and sexual impotence.

Under Finasteride group 2, compared with placebo Group 1, events were: erectile dysfunction, libido decrease and/or abnormal ejaculation.

Under combination group 4, two side effects were reported those which were manifested by abnormal ejaculation.

Upon completing the study, 95% of patients from Alfuzosine group 3 discontinued the treatment.

Upon completing the study, 45% of patients from Finasteride group 2 discontinued the treatment.

No patient from the group 4 corresponding to Finasteride/Alfuzosine combination cancelled the treatment.

### Conclusions:

The clinical trial showed that Alfuzosine, Finasteride and a combination of both drug substances reduced the clinical risk of Benign Prostatic Hyperplasia progression, which was defined when comparing against placebo, which resulted in symptom worsening such as: acute urinary retention, incontinence, urinary tract infection or renal failure. A combination of drug substances in a single dose unit where a lower concentration of α-adrenergic receptor antagonist agent was included was significantly more effective when drug substances were independently administered and at higher therapeutic doses.

Combination therapy mostly reduced the BPH clinical progression unlike when administered drug substances independently, thus reducing a risk of manifesting an acute urinary retention in long-term and a need of invasive therapy related with BPH. Our results indicate that long-term therapy with a combination of Finasteride/ Alfuzosine is safe and effective and also well-tolerated.

## Claims

1. Pharmaceutical composition **characterized by** comprising a synergic combination of Finasteride and Alfuzosine, together with pharmaceutically acceptable excipients, which are formulated in a single dosage unit to be orally administered once a day, **characterized in that** Finasteride is present in the formulation in a concentration of 5 mg and Alfuzosine is present in the formulation in a concentration of 5.0 mg per dose unit.

2. Pharmaceutical composition in accordance with claim 1, **characterized in that** it is orally administered in a tablet form.

3. Use of the pharmaceutical composition in accordance with claims 1 to 2, for the manufacture of a medicament useful for treatment of Benign Prostatic Hyperplasia as well as for a relief of the symptoms manifested by said disease.

## Patentansprüche

1. Pharmazeutische Zusammensetzung, **dadurch gekennzeichnet, dass** sie eine synergetische Kombination aus Finasterid und Alfuzosin zusammen mit pharmazeutisch annehmbaren Hilfstoffen umfasst, welche in einer Einzeldosierungseinheit zur einmal täglichen oralen Verabreichung formuliert sind, **dadurch gekennzeichnet, dass** Finasterid in der Formulierung in einer Konzentration von 5 mg vorliegt und Alfuzosin in der Formulierung in einer Konzentration von 5,0 mg je Dosiseinheit vorliegt.

2. Pharmazeutische Zusammensetzung nach Anspruch 1, **dadurch gekennzeichnet, dass** sie oral in Tablettenform verabreicht wird.

3. Verwendung der pharmazeutischen Zusammensetzung nach Anspruch 1 bis 2 zur Herstellung eines Medikamentes, welches sich zu der Behandlung der benignen Prostatahyperplasie sowie zur Linderung der durch diese Krankheit ausgebildeten Symptome eignet.

## Revendications

1. Composition pharmaceutique **caractérisée en ce qu'**elle comprend une combinaison synergique de Finastéride et d'Alfuzosine, conjointement aux excipients pharmaceutiquement acceptables, qui sont formulés en une seule unité de dosage destinée à être administrée par voie orale une fois par jour, **caractérisée en ce que** le Finastéride est présent dans la formulation à une concentration de 5 mg et l'Alfuzodine est présent dans la formulation à une concentration de 5,0 mg par unité de dosage.

2. Composition pharmaceutique selon la revendication 1, **caractérisée en ce qu'**elle est administrée par voie orale sous la forme de comprimés.

3. Utilisation de la composition pharmaceutique selon les revendications 1 à 2, pour la fabrication d'un médicament utile pour le traitement de l'hypertrophie bénigne de la prostate ainsi que pour le soulagement des symptômes manifestés par ladite maladie.
